(19) Europäisches Patentamt
European Patent Office
Office européen des brevets

(11) **EP 1 453 855 B1**

(12) **EUROPÄISCHE PATENTSCHRIFT**

(45) Veröffentlichungstag und Bekanntmachung des Hinweises auf die Patenterteilung:
**18.02.2009 Patentblatt 2009/08**

(21) Anmeldenummer: **02792890.2**

(22) Anmeldetag: **04.12.2002**

(51) Int Cl.:
*C07K 14/47* (2006.01)     *C07K 7/08* (2006.01)
*G01N 33/68* (2006.01)     *C12P 21/02* (2006.01)
*A61K 47/48* (2006.01)     *A23B 4/20* (2006.01)
*A61P 31/04* (2006.01)

(86) Internationale Anmeldenummer:
**PCT/EP2002/013724**

(87) Internationale Veröffentlichungsnummer:
**WO 2003/048201 (12.06.2003 Gazette 2003/24)**

(54) **ANTIMIKROBIELLE BOLISINPEPTIDE**

ANTIMICROBIAL BOLISIN PEPTIDES

PEPTIDES BOLISINE ANTIMICROBIENS

(84) Benannte Vertragsstaaten:
**AT BE BG CH CY CZ DE DK EE ES FI FR GB GR IE IT LI LU MC NL PT SE SI SK TR**

(30) Priorität: **07.12.2001 EP 01129094**

(43) Veröffentlichungstag der Anmeldung:
**08.09.2004 Patentblatt 2004/37**

(73) Patentinhaber: **IPF Pharmaceuticals GmbH 30625 Hannover (DE)**

(72) Erfinder:
• **LIEPKE, Cornelia 30625 Hannover (DE)**
• **BAXMANN, Susann 31275 Lehrte (DE)**
• **ADERMANN, Knut 30177 Hannover (DE)**

(74) Vertreter: **von Kreisler Selting Werner Patentanwälte P.O. Box 10 22 41 50462 Köln (DE)**

(56) Entgegenhaltungen:
WO-A-01/94386     WO-A-97/35877
DE-A- 4 444 753

## Beschreibung

Die Erfindung betrifft Peptide mit antimikrobieller Aktivität hoher Wirksamkeit, Verfahren zu ihrer Herstellung sowie ihre Verwendung.

Es ist bekannt, dass natürlich vorkommende Peptide antibiotisch wirksam sein können. So beschreibt die WO 97/35877 das Vorkommen und die Aufreinigung von antibiotischen Peptiden aus Kuhmilch und die PCT/EP 01/06518 die Gewinnung von antimikrobiellen Peptiden aus humanem Plazentaextrakt und aus bovinem Thymusextrakt sowie ihre Anwendung.

Eines der aus bovinem Thymusextrakt gereinigten Peptide ist Bolisin (PCT/EP 01/06518, Seq ID No. 5). Bolisin ist ein 17 Aminosäuren umfassendes Fragment des bovinen mitochondrialen Proteolipids und inhibiert das Wachstum von pathogenen Mikroorganismen in salzarmen Nährmedien.

DE 44 44 753 A1 offenbart ein Verfahren zur Produktion eines antibiotisch wirksamen Peptid-Präparates aus der Kuhmilch. Die Gewinnung dieser Peptide kann entweder durch eine gezielte Verarbeitung von Vorzugsmilch erfolgen, oder sie erfolgt über die chemische Peptid-Synthese.

Ferner offenbart WO 97/35877 Peptide mit der Aminosäuresequenz $H_2N-X_1-R-X_3-X_2-COOH$, wobei $X_1$ entweder Null ist oder $X_1$ und/oder $X_2$ Reste mit mindestens fünf Aminosäuren sind.

Der Erfindung liegt die Aufgabe zugrunde, auf der Basis des Bolisin Peptide bereitzustellen, die unter verschiedenen Bedingungen eine besonders hohe spezifische Wirksamkeit aufweisen. Aufgabe der Erfindung ist es auch, Peptide bereitzustellen, die das Wachstum von Mikroorganismen in Umgebungen mit physiologischen Salzkonzentration inhibieren und gleichzeitig eine möglichst geringe hämolytische Aktivität aufweisen.

Überraschenderweise wird die Aufgabe gelöst durch Bereitstellung von Peptiden der Sequenz

$$R_1 - ZX_3X_3X_1BX_3RX_2X_3X_4BRX_2BX_3X_3B - R_2,$$

wobei

$R_1$ eine Aminogruppe ($NH_2$), eine Aminosäure oder ein Peptid mit bis zu 10 Aminosäuren und $R_2$ COOH, $CONH_2$, eine Aminosäure oder ein Peptid mit bis zu 10 Aminosäuren;

Z eine aromatische Aminosäure (W, Y, F), bevorzugt Tyrosin (Y) oder eine am Aromaten ein- oder mehrfach halogenierte Aminosäure, bevorzugt Tyrosin (Y);

$X_1$ Arginin (R) oder eine aromatische Aminosäure (W,Y,F), bevorzugt Tryptophan (W) oder eine am Aromaten ein- oder mehrfach halogenierte Aminosäure, bevorzugt Tryptophan;

$X_2$ Serin (S), eine basische Aminosäure (R, K), bevorzugt Arginin (R), oder eine aromatische Aminosäure (W,Y,F), bevorzugt Tryptophan (W) oder eine am Aromaten ein- oder mehrfach halogenierte Aminosäure, bevorzugt Tryptophan;

$X_3$ Threonin (T), eine hydrophobe Aminosäure (I, V, A, L) oder Arginin (R);

$X_4$ Asparaginsäure (D), Prolin (P), eine basische Aminosäure (K, R), bevorzugt Arginin (R) oder eine aromatische Aminosäure (W,Y,F), bevorzugt Tryptophan (W) oder eine am Aromaten ein- oder mehrfach halogenierte Aminosäure, bevorzugt Tryptophan und

B eine basische Aminosäure (K,R) ist.

Gegenstand der Erfindung sind auch Derivate und/oder Fragmente der erfindungsgemässen Peptide mit antimikrobieller Aktivität, insbesondere die amidierten, acylierten, acetylierten, alkylierten, sulfatierten, phosphorylierten, halogenierten, insbesondere an aromatischen Aminosäureseitenketten halogenierten, glycosylierten, oxydierten, durch Veresterung oder Laktonformation modifizierten und/oder zyklisierten Derivate und/oder Fragmente. Halogenierte Peptide sind vorzugsweise an aromatischen Resten von Aminosäureseitenketten halogeniert. Die Aminosäuren sind durch den Ein-Buchstabencode bezeichnet.

Dabei umfassen die Peptide nicht die Aminosäuresequenz

$R_3$-YIVYKIRSADKRSKALK-$R_4$, worin $R_3$ eine Aminogruppe ($NH_2$), eine Aminosäure oder ein Peptid mit bis zu 50 Aminosäuren und $R_4$ COOH, $CONH_2$, eine Aminosäure oder ein Peptid mit bis zu 50 Aminosäuren darstellt, sowie nicht die amidierten, acetylierten, sulfatierten, phosphorylierten, glycosylierten, oxydierten Derivate und weiterhin Fragmente mit antimikrobiellen Eigenschaften, die aus der Aminosäuresequenz abgeleitet werden, insbesondere solche Fragmente, die durch konservativen Austausch oder Deletion von Aminosäuren erhalten wurden.

Vorzugsweise weisen die erfindungsgemässen Peptide die Sequenzen

$R_1$ - $ZIX_3X_1BX_3RX_2ADBRX_2BALB$ - $R_2$ (Sequenz ID No.: 2,4,8,12,13,15)

$R_1$ - $ZIX_3X_1BX_3RX_2APBRX_2BALB$ - $R_2$ (Sequenz ID No.: 11)

$R_1$ - $ZIX_3X_1BX_3RX_2ABBRX_2BALB$ - $R_2$ (Sequenz ID No.: 1, 3, 5, 6, 7, 9, 10, 18, 21-24, 28, 29, 31, 32, 35, 38, 41, 45, 47, 48, 51)

$R_1$ - $ZIX_3X_1BX_3RX_2AZBRX_2BALB$ - $R_2$ (Sequenz ID No.: 14, 16, 17, 19, 20, 25-27, 30, 33, 34, 36, 37, 42-44, 46, 49, 50, 52, 53, 54)

$R_1$ - $ZIX_3X_1BX_3RX_2TZBRX_2BALB$ - $R_2$ (Sequenz ID No.: 55)

auf, wobei $X_3$ eine hydrophobe Aminosäure (I, V) oder Arginin (R) ist und $R_1$, $R_2$, Z, $X_1$, $X_2$ und B die bereits oben aufgeführten Bedeutungen haben. Bevorzugte Peptide der Erfindung sind in der Tabelle 1 aufgeführt. Gegenstand der Erfindung sind auch deren Derivate und/oder Fragmente mit antimikrobieller Aktivität, insbesondere die amidierten, acylierten, acetylierten, alkylierten, sulfatierten, phosphorylierten, halogenierten, glycosylierten, oxydierten, durch Veresterung oder Laktonformation modifizierten und/oder = zyklisierten Derivate und/oder Fragmente.

Die Erfindung beinhaltet die durch Veresterung oder Laktonformation modifizierten Peptide sowie die erfindungsgemässen Peptide, die an einer freien Aminogruppe mit aktiviertem Polyoxyalkylenglycol oder einem Konjugat aus einem Polyoxyalkylenglycol und z. B. einer Fettsäure umgesetzt werden. Bevorzugt sind dabei Fettsäuren mit einer Kettenlänge von 12-18 Kohlenstoffatomen und Polyoxyethylen als Polyoxyalkylenglycol mit einer Kettenlänge von 2 bis 100. Weiterhin bevorzugt sind aktivierte Polysorbatester und deren Derivate. Die Peptidkonjugate werden zur Verbesserung der pharmakologischen Eigenschaften der Peptide hergestellt.

Neben den erwähnten Derivaten können die Peptide auch statt der natürlichen L-Aminosäuren die korrespondierenden D-Aminosäuren sowie Imino-Aminosäuren und seltene Aminosäuren, wie Hydroxylysin, Homoserin und Ornithin aufweisen. Weiterhin beinhaltet die Erfindung die Retro-, Inverso- und Retro-Inverso-Peptide. Als Retropeptide werden die erfindungsgemässen Peptide mit reverser Aminosäuresequenz bezeichnet, Inversopeptide stellen die erfindungsgemässen Peptide, die aus D-Aminosäuren bestehen und Retro-Inverso-Peptide diejenigen Peptide dar, die eine reverse Aminosäuresequenz, gebildet aus D-Aminosäuren, aufweisen.

Gegenstand der Erfindung sind auch Peptidmimetika der erfindungsgemäßen Peptide. Diese sind durch Modifikation einer oder mehrerer Peptidbindungen, zum Beispiel durch eine reverse Peptidbindung oder durch eine Esterbindung charakterisiert.

Bei den erfindungsgemässen Bolisinanaloga handelt es sich um kationische antibiotisch wirksame Peptide. Die Bolisinanaloga enthalten wenigstens 9 und höchstens 37 Aminosäurereste, bevorzugt nicht mehr als 20 Aminosäurereste.

Die erfindungsgemässen Peptidanaloga von Bolisin weisen eine deutlich erhöhte antimikrobielle Wirksamkeit auf. Die in dieser Erfindung beschriebenen Peptide inhibieren auch in Nährmedien, die eine physiologische Salzkonzentration aufweisen, effektiv das Wachstum von pathogenen Keimen und sind daher im Gegensatz zum natürlich vorkommenden Bolisin für eine Vielzahl von Anwendungen nutzbar. Auch human pathogene Keime mit Antibiotikaresistenz werden bereits bei geringen Peptidkonzentrationen abgetötet.

Als Fragmente kommen insbesondere N- oder C-terminal verkürzte Peptide in Frage, aber auch Peptide, in denen einzelne Aminosäuren deletiert sind, vorzugsweise nicht mehr als 10 % der Aminosäuren. Diese Fragmente sind insoweit Gegenstand der Erfindung, als sie antimikrobielle Eigenschaften aufweisen. Antimikrobielle Eigenschaften des Fragmentes bedeutet, dass das Fragment mindestens 50 % der Aktivität des Stammpeptides hat, also maximal die doppelte Hemmkonzentration benötigt. Dabei werden als Testmikroorganismen entweder *Staphylococcus aureus* oder *Escherichia coli* verwendet, je nachdem, gegen welchen Mikroorganismus das Peptid eine bessere Wirksamkeit aufweist. Die Mikroorganismen sind wie folgt charakterisiert:

*Staphylococcus aureus:* Gram-positive Kokken, ATCC 25923

*Escherichia coli:* Gram-negative Stäbchen, ATCC 25922

Die Erfindung umfasst auch Nucleinsäuren, die für die erfindungsgemässen Peptide kodieren, und Vektoren und Plasmide, die solche Nucleinsäuren enthalten.

Gegenstand der Erfindung ist auch ein Arzneimittel, das ein oder mehrere efindungsgemässe Peptide enthält.

Die erfindungsgemässen Peptide eignen sich zur Behandlung von durch mikrobielle Fehlbesiedlungen bedingten Erkrankungen wie Infektionen, Entzündungen, mikrobiell induzierten Tumoren, mikrobiell bedingten degenerativen Erkrankungen, Durchfallerkrankungen, Koliken, Abweichungen der Mund-, Darm- und Vaginalflora, Karies, Sepsis, toxische Schockzustände. Die mikrobielle Fehlbesiedlung kann beispielsweise durch Bakterien, Pilze, Hefen, Protisten, Viren, Mycoplasmen, Filarien und/oder Plasmodien bedingt sein. Sie können sowohl bei akuten als auch bei chronischen Erkrankungen von Menschen und Tieren eingesetzt werden.

Die erfindungsgemässen Peptide werden bevorzugt in Arzneimittelzubereitungen verwendet. Die Arzneimittelzubereitung enthält eines oder mehrere der erfindungsgemässen Peptide bzw. ein physiologisch verträgliches Salz der Peptide. Arzneimittelzubereitungen können pharmazeutisch übliche Hilfsstoffe, die z. B. einen Beitrag zur Löslichkeit, Stabilität oder Sterilität des Arzneimittels leisten oder den Wirkungsgrad der Aufnahme in den Körper erhöhen, enthalten. Weiterhin können weitere antibiotische Substanzen enthalten sein, zum Beispiel aus der Gruppe der Penicilline, Cephalosporine, Carbacepheme, Cephamycine, Carbapeneme, Quinolone, Tetracycline, Aminoglycoside, Makrolide, Glycopeptide, Chloramphenicole, Glycylcycline, Licosamide, Fluoroquinolone und Peptidantibiotika.

Die Form und Zusammensetzung des Arzneimittels, welches die Peptide enthält, richtet sich nach der Art der Verabreichung. Bevorzugt werden galenische Zubereitungen eingesetzt und Applikationsformen gewählt, bei denen die Peptide undegradiert an den Wirkort gelangen. Die Peptide können enteral, parenteral, intranasal, oral und mittels Inhalation verabreicht werden. Vorzugsweise werden die Peptide zu einem Injektionspräparat, entweder als Lösung oder als Lyophilisat zur Auflösung unmittelbar vor Gebrauch, konfektioniert. Die Peptide können zum Beispiel intravenös injiziert, intraperitoneal injiziert oder implantiert, subcutan injiziert oder implantiert, intradermal injiziert, intramusculär injiziert oder implantiert, intrathecal injiziert, inhaliert (Aerosol, Spray) oder topisch (z. B. Cremes, Salben, Augentropfen, Ohrtropfen, Shampoos) appliziert werden. Bolisinanaloga können lokal als Injektion, Tropfen, Spray, Tabletten, Zäpfchen, Creme, Salben, Gel etc. verabreicht werden. Es besteht die Möglichkeit der Verabreichung als Bolus oder mehrfach über eine Zeitperiode.

Die zu verabreichende Dosis der Peptide hängt von der Indikation und der Anwendung bestimmter Derivate ab. Vorzugsweise werden die erfindungsgemässen Peptide in Mengen von 0,1 bis 100 mg/kg Körpergewicht eingesetzt.

Gegenstand der Erfindung ist auch ein Diagnostikmittel, das erfindungsgemässe Peptide oder Nucleinsäuren enthält.

Die erfindungsgemässen Peptide können auch als Zusatzstoffe für Lebensmittel verwendet werden. Sie können bei der Herstellung von Lebensmitteln als Hilfsstoffe, insbesondere bei Lebensmitteln, die durch Gärung und andere bakterielle Prozesse hergestellt werden, verwendet werden. Bei den patentgemäßen Präparaten handelt es sich um natürliche Konservierungsmittel. Weitere Möglichkeiten der Anwendung der erfindungsgemässen Peptide sind die Nutzung als Desinfektionsmittel für alle Situationen, in denen Mikroorganismen unerwünscht sind. Beispiele sind die Anwendung zur Oberflächendesinfektion oder als Beschichtung von Medizinprodukten, Luftfiltern und Bedarfsgegenständen wie z. B. Kleidung und Schmuck. Unter Medizinprodukten werden alle medizinischen Hilfsstoffe verstanden, die am Patienten Anwendung finden, wie z. B. Implantate, Prothesen, Stents, Tuben, Kanülen, Katheter, synthetische Gefäßsysteme, künstliche Herzklappen und Nadeln. Als Medizinprodukte werden weiterhin auch Behälter zur Aufbewahrung von Blut oder Blutbestandteilen, sowie Behälter zum Organtransport etc. verstanden. Weiterhin ist der Einsatz in der Wasseraufbereitung oder als Bestandteil von Shampoos und Seifen, als Lebensmittelzusatzstoffe, kosmetische Konservierungsstoffe, Zusatzstoffe zur Konservierung von Zellkulturmedien etc. sowie Pflanzenschutzmittel möglich.

<u>Ausführungsbeispiele:</u>

<u>Chemische Synthese von Bolisinanaloga</u>

Die Peptidsynthese von Bolisinanaloga basiert auf dem Standardverfahren der Festphasen Fmoc Strategie. Zur automatisierten Synthese wurde ein ABI 433 A Synthesizer (Applied Biosystems, Weiterstadt, Deutschland) benutzt. Als Festphase diente ein Fmoc-Lys(boc) Wang Harz. Während der Peptidsynthese wurden die einzelnen Kopplungsschritte kontrolliert, um sicherzustellen, dass jede Aminosäure in hoher Ausbeute inkorporiert wurde. Peptide wurden durch 94% Trifluoressigsäure (TFA), 3% Ethandithiol und 3% Wasser vom Harz abgespalten und anschließend chromatographisch gereinigt.

Zur chromatographischen Reinigung wurde die reverse Phase (RP) HPLC angewendet. Als feste Phase diente eine präparative RP C18 Säule (PrepPAK 500 C18, 47x300 mm), als Elutionsmittel Acetonitril (Fließmittel A: 0.07 % TFA in Wasser; Fließmittel B: 80 % Acetonitril / 0.07 % TFA in Wasser). Bei einer Fließgeschwindigkeit von 40 ml/min wurden die Peptide im Gradientenverfahren (Anstieg von 0.5 % Fließmittel B /min im Elutionsmittel) von der Festphase eluiert und bei einer Wellenlänge von 215 nm detektiert.

Die synthetischen Peptide wurden massenspektrometrisch analysiert. Die Reinheit der Peptide wurde über Kapillarzonenelektrophorese und analytische reverse Phase HPLC kontrolliert (Reinheit ≥ 95 %). Die Aminosäuresequenz der Peptide wurde über automatisierten Edman-Abbau überprüft.

Die Aminosäuresequenzen der synthetisierten erfindungsgemässen Bolisinanaloga sind unter Verwendung des Einbuchstabenkodes in Tabelle 1 angegeben.

**Tabelle 1:**

| Name | Seq ID NO | Sequenz |
|---|---|---|
| BOL-6 | 1 | YIVYKIRSARKRSKALK |
| BOL-7 | 2 | YIVYKIRSADKRRKALK |
| BOL-11 | 3 | YIVYKRRSARKRSKALK |
| BOL-12 | 4 | YIVYKRRSADKRRKALK |
| BOL-13 | 5 | YIVYKIRSARKRRKALK |
| BOL-14 | 6 | YIRYKIRSARKRRKALK |
| BOL-15 | 7 | YIVYKRRSARKRRKALK |
| BOL-16 | 8 | YIRYKRRSADKRRKALK |
| BOL-17 | 9 | YIRYKIRSARKRRKALK |
| BOL-18 | 10 | YIRYKRRSARKRRKALK |
| BOL-21 | 11 | YIVYKIRSAPKRSKALK |
| BOL-25 | 12 | YIVWKIRSADKRSKALK |
| BOL-27 | 13 | YIVYKIRWADKRSKALK |
| BOL-29 | 14 | YIVYKIRSAWKRSKALK |
| BOL-30 | 15 | YIVYKIRSADKRWKALK |
| BOL-44 | 16 | YIVYKIRSAWKRRKALK |
| BOL-45 | 17 | YIVYKIRSAWKRWKALK |
| BOL-46 | 18 | YIVYKIRSARKRWKALK |
| BOL-47 | 19 | YIVYKIRWAWKRSKALK |
| BOL-48 | 20 | YIVYKIRWAWKRRKALK |
| BOL-49 | 21 | YIVYKIRWARKRSKALK |
| BOL-50 | 22 | YIVYKIRWARKRRKALK |
| Box-51 | 23 | YIVYKIRWARKRWKALK |
| BOL-52 | 24 | YIVYKIRRARKRRKALK |
| BOL-53 | 25 | YIVYKIRRAWKRSKALK |
| BOL-54 | 26 | YIVYKIRRAWKRRKALK |
| BOL-55 | 27 | YIVYKIRRAWKRWKALK |
| BOL-56 | 28 | YIVYKIRSAKKRKKALK |
| BOL-57 | 29 | YIVYKIRSAKKRWKALK |
| BOL-58 | 30 | YIVYKIRSAWKRKKALK |
| BOL-59 | 31 | YIVYKIRWAKKRKKALK |
| Bo-60 | 32 | YIVYKIRWAKKRWKALK |
| BOL-61 | 33 | YIVYKIRWAWKRKKALK |
| BOL-67 | 34 | YIVYKIRSAWKRWKAL |

(fortgesetzt)

| Name | Seq ID NO | Sequenz |
|---|---|---|
| BOL-68 | 35 | YIVYKIRSARKRWKAL |
| BOL-69 | 36 | YIVYKIRSAWKRRK |
| BOL-70 | 37 | YIVYKIRSAWKRWK |
| BOL-71 | 38 | YIVYKIRSARKRWK |
| BOL-72 | 39 | YIVYKIRWARKRRKAL |
| BOL-73 | 40 | YIVYKIRWARKRRK |
| BOL-74 | 41 | YIVWKIRSARKRRKALK |
| BOL-75 | 42 | YIVWKIRSAWKRSKALK |
| BOL-79 | 43 | YIVRKIRSAWKRWKALK |
| BOL-80 | 44 | YIVRKIRSAWKRRKALK |
| BOL-81 | 45 | YIVWKIRSARKRWKALK |
| BOL-82 | 46 | YIVWKIRSAWKRWKALK |
| BOL-83 | 47 | YWRKIRSARKRRKALK |
| BOL-84 | 48 | KYIVYKIRSARKRRKALK |
| BOL-85 | 49 | KYIVYKIRSAWKRSKALK |
| BOL-86 | 50 | KYIVYKIRSAWKRRKALK |
| BOL-87 | 51 | KYIVYKIRWARKRRKALK |
| BOL-91 | 52 | YIVYKIRFAFKRSKAL |
| BOL-92 | 53 | YIVYKIRFAFKRRKAL |
| BOL-93 | 54 | YIVYKIRWAWKRSKAL |
| BOL-97 | 55 | YIVYKIRWTWKRSKAL |
| BOL-103 | 56 | KLAKRRKWAWRIKYVIY |

Untersuchung der antimikrobiellen Wirksamkeit von Bolisinanaloga

Die Bolisinanaloga der vorliegenden Erfindung wurden mit Hilfe verschiedener biologischer Tests auf ihre Eignung als antibiotische Therapeutika untersucht. Dazu wurden die chemisch synthetisierten Peptide auf ihre antimikrobielle Wirksamkeit gegen drei Gram-positive und drei Gram-negative Bakterienstämme sowie gegen eine Hefe untersucht.
Folgende Mikroorganismen wurden verwendet:

| | |
|---|---|
| **Gram-positiv:** | *Staphylococcus aureus* ATCC 25923 |
| | *Enterococcus faecalis* ATCC 51299 (Vancomycin resistent) |
| | *Streptococcus pneumoniae* DSM 11865 (Penicillin resistent) |
| **Gram-negativ:** | *Escherichia coli* ATCC 25922 |
| | *Pseudomonas aeruginosa* ATCC 9027 |
| | *Klebsiella pneumoniae* ATCC 10031 |
| **Hefe:** | *Candida albicans* ATCC 10231 |

Die antibiotische Aktivität von Bolisinanaloga wurde mit Hilfe der Bestimmung der minimalen Inhibitionskonzentration untersucht. Die minimale Inhibitionskonzentration eines Analogen bezeichnet die geringste Peptidkonzentration, die erforderlich ist, um das Wachstum von Mikroorganismen nach einer Inkubationszeit von $18 \pm 2$ h vollständig zu inhibieren. Diese Methode erlaubt eine quantitative Aussage über die antimikrobielle Potenz der Peptide. Diese Untersuchungen wurden in Anlehnung an die von dem NCCLS (National Committee for Clinical Laboratory Standards) herausgegebenen Empfehlungen (M7-A3) unter Anwendung des Verdünnungstest in Flüssigmedium durchgeführt, wobei chemisch synthetisierte Peptide (Peptidsequenzen gemäß Anspruch 1-3) verwendet wurden. Als Testmedium wurde gemäß den NCCLS Empfehlungen Mueller Hinton Broth verwendet, soweit nicht anderweitig gekennzeichnet. Tabelle 2 zeigt die im Vergleich zu dem Ursprungspeptid Bolisin deutlich verbesserte antimikrobielle Aktivität der Peptidanaloga. Die minimalen Hemmkonzentrationen sind in [µg/ml] angegeben.

**Tabelle 2:**

| Mikroorganismen | 1 | 2 | 3 | 4 | 5 | 6 | 7 |
|---|---|---|---|---|---|---|---|
| Bolisin* | >300 | >300 | >300 | >300 | >300 | >300 | nd |
| Bolisin | >300 | >300 | >300 | >300 | >300 | >300 | >300 |
| Bolisin** | 25 | 300 | 300 | 6.25 | 150 | 150 | nd |
| BOL-6* | 37.5 | 200 | 150 | 37.5 | (9.375) | 37.5 | nd |
| BOL-7* | 75 | >300 | >300 | 150 | (75) | 300 | nd |
| BOL-11* | 37.5 | >300 | >300 | 37.5 | (150) | 150 | nd |
| BOL-12* | 18.75 | >300 | >300 | 18.75 | (300) | 200 | nd |
| BOL-13* | 18.75 | 75 | 150 | 18.75 | (9.375) | 18.75 | nd |
| BOL-14* | 50 | >300 | >300 | 37.5 | (150) | 75 | nd |
| BOL-15* | 18.75 | >300 | >300 | 18.75 | (150) | 75 | nd |
| BOL-16* | 100 | >300 | >300 | 75 | (300) | 150 | nd |
| BOL-17* | 37.5 | 200 | 300 | 37.5 | 200 | 9.375 | nd |
| BOL-18* | 18.75 | >300 | >300 | 18.75 | 300 | 37.5 | nd |
| BOL-21* | 75 | >300 | >300 | 150 | (150) | 200 | nd |
| BOL-25* | 150 | >300 | >300 | 150 | (150) | 200 | nd |
| BOL-27* | 75 | 300 | 300 | 75 | (150) | 150 | nd |
| BOL-29* | 9.375 | 75 | 150 | 9.375 | (18.75) | 50 | nd |
| BOL-30* | 100 | >300 | >300 | 150 | (300) | 300 | nd |
| BOL-44 | 150 | >300 | 200 | 200 | (75) | 75 | 37.5 |
| BOL-45 | 18.75 | 100 | 100 | 12.5 | (25) | 4.7 | 18.75 |
| BOL-46 | 300 | 200 | 200 | 200 | (100) | 150 | 150 |
| BOL-47 | 18.75 | 25 | 18.75 | 18.75 | (25) | 9.375 | 18.75 |
| BOL-48 | 9.375 | 12.5 | 9.375 | 18.75 | 200 (6.5) | 4.7 | 9.375 |
| BOL-49 | 75 | 25 | 18.75 | 50 | (12.5) | 18.75 | 18.75 |
| BOL-50 | 37.5 | 25 | 18.75 | 50 | (6.25) | 9.375 | 9.375 |
| BOL-51 | 37.5 | 25 | 12.5 | 50 | 300 (25) | 18.75 | (18.75) |
| BOL-52 | 300 | 200 | 200 | >300 | (37.5) | 75 | (37.5) |
| BOL-53 | 150 | 200 | 200 | 200 | (75) | 100 | (37.5) |
| BOL-54 | 200 | 100 | 75 | 100 | (25) | 18.75 | 18.75 |
| BOL-55 | 12.5 | 50 | 37.5 | 12.5 | (9.375) | 3.125 | 9.375 |
| BOL-56 | >300 | >300 | >300 | >300 | (200) | 300 | (300) |
| BOL-57 | >300 | >300 | >300 | >300 | (200) | 300 | 300 |
| BOL-58 | >300 | >300 | 300 | >300 | (100) | 100 | (75) |

(fortgesetzt)

| Mikroorganismen | 1 | 2 | 3 | 4 | 5 | 6 | 7 |
|---|---|---|---|---|---|---|---|
| BOL-59 | 50 | 50 | 50 | 150 | (18.75) | 18.75 | (9.375) |
| BOL-60 | 50 | 25 | 50 | 100 | (18.75) | 18.75 | (18.75) |
| BOL-61 | 37.5 | 25 | 25 | 25 | 300 (75) | 18.75 | 9.375 |
| BOL-67 | 25 | 100 | 100 | 25 | (50) | 37.5 | 18.75 |
| BOL-68 | 200 | 200 | 300 | 200 | (200) | 300 | 200 |
| BOL-69 | 300 | 150 | 200 | 150 | (200) | 300 | 37.5 |
| BOL-70 | 100 | 150 | 150 | 100 | (100) | 75 | 37.5 |
| BOL-71 | 200 | 200 | 300 | 300 | (200) | 300 | 300 |
| BOL-72 | 75 | 25 | 18.75 | 100 | (12.5) | 18.75 | 18.75 |
| BOL-73 | 37.5 | 25 | 18.75 | 50 | (9.375) | 18.75 | 9.375 |
| BOL-74 | 300 | 150 | 150 | 300 | (12.5) | 18.75 | 75 |
| BOL-75 | 150 | 200 | 150 | 75 | (37.5) | 75 | 37.5 |
| BOL-79 | 9.375 | >300 | 300 | 3.125 | (4.7) | 2.35 | 4.7 |
| BOL-80 | >300 | >300 | >300 | >300 | (18.75) | 150 | 37.5 |
| BOL-81 | 300 | 150 | 75 | 300 | (18.75) | 25 | 75 |
| BOL-82 | 18.75 | 75 | 37.5 | 18.75 | (9.375) | 12.5 | 9.375 |
| BOL-83 | >300 | >300 | >300 | >300 | >300 | >300 | 300 |
| BOL-84 | >300 | >300 | 300 | >300 | (75) | 75 | 37.5 |
| BOL-85 | >300 | 300 | 300 | >300 | (37.5) | 150 | 75 |
| BOL-86 | 75 | 200 | 150 | 150 | (18.75) | 50 | 18.75 |
| BOL-87 | 9.375 | 37.5 | 9.375 | 18.75 | (9.375) | 9.375 | 9.375 |
| BOL-91 | 25 | 37.5 | 37.5 | 25 | (150) | 18.75 | nd |
| BOL-92 | 18.75 | 18.75 | 25 | 18.75 | (37.5) | 12.5 | nd |
| BOL-93 | 25 | 37.5 | 25 | 25 | (37.5) | 25 | nd |
| BOL-97 | 12.5 | 18.75 | 18.75 | 18.75 | (37.5) | 18.75 | nd |
| BOL-103 | 18.75 | 37.5 | 37.5 | 18.75 | (9.4) | 18.75 | nd |

*Testmedium: halbkonzentrierte Mueller Hinton Broth
**Testmedium: 3g/l Tryptic Soy Broth (ionenschwaches Medium)

Werte in Klammern bezeichnen eine 75%ige Inhibition des Mikroorganismenwachsstums bei der angegebenen Peptidkonzentration. Eine komplette Wachstumshemmung konnte in diesem Fall nicht erreicht werden. Zur Bestimmung der minimalen Inhibitionskonzentration wurden folgende Mikroorganismen verwendet:

1. *Staphylococcus aureus* ATCC 25923

2. *Enterococcus faecalis* ATCC 51299 (Vancomycin resistent)

3. *Streptococcus pneumoniae* DSM 11865 (Penicillin resistent)

4. *Escherichia coli* ATCC 25922

5. *Pseudomonas aeruginosa* ATCC 9027

6. *Klebsiella pneumoniae* ATCC 10031

7. *Candida albicans* ATCC 10231

Die erfindungsgemässen Peptide inhibieren effektiv das Wachstum sowohl von Gram-positiven und Gram-negativen Bakterien als auch von Hefen. Human pathogene Erreger, wie *Staphylococcus aureus, Escherichia coli, Pseudomonas aeruginosa* und *Klebsiella pneumoniae* werden effektiv abgetötet. Von besonderer Bedeutung ist die sehr effektive Wachstumshemmung von Bakterien mit bereits bestehender Antibiotikaresistenz wie z. B. *Streptococcus pneumoniae* (Penicillin resistent) und *Enterococcus faecalis* (Vancomycin resistent). Die Wirkpotenz der Bolisinanaloga ist um ein Vielfaches besser als die des Ursprungspeptides Bolisin.

Untersuchung der Wirksamkeit bei erhöhter Salzkonzentration

Auch für diese Untersuchungen wurde die minimale Inhibitionskonzentration der Peptide gegen Gram-positive und Gram-negative Mikroorganismen (Mikroorganismen 1-6) bestimmt. Als Testmedium wurde Tryptic Soy Broth (3 g/l) mit einem Zusatz von Salz in physiologischer Konzentration (150 mM Natriumchlorid) verwendet. Tabelle 3 zeigt die gegenüber dem Ursprungspeptid Bolisin ebenfalls deutlich verbesserte antimikrobielle Aktivität der Peptidanaloga in salzhaltigem Medium.

**Tabelle 3:**

| Mikroorganismen | 1 | 2 | 3 | 4 | 6 |
|---|---|---|---|---|---|
| Bolisin | >300 | >300 | >300 | >300 | >300 |
| BOL-44 | 200 | (300) | >300 | 150 | >300 |
| BOL-45 | 37.5 | 200 | 200 | 75 | 4.7 |
| BOL-46 | >300 | >300 | >300 | >300 | >300 |
| BOL-47 | 18.75 | 50 | 37.5 | 25 | 9.375 |
| BOL-48 | 18.75 | 18.75 | 25 | 9.375 | 9.375 b |
| BOL-49 | 75 | 75 | 100 | 50 | 75 |
| BOL-50 | 37.5 | 50 | 50 | 37.5 | 37.5 |
| BOL-51 | 75 | 37.5 | 37.5 | 37.5 | 25 |
| BOL-52 | >300 | >300 | >300 | >300 | (300) |
| BOL-53 | 300 | >300 | >300 | 200 | >300 |
| BOL-54 | 300 | 300 | 300 | 200 | (200) |
| BOL-55 | 18.75 | 150 | 150 b | 12.5 | 4.7 |
| BOL-56 | >300 | >300 | >300 | >300 | >300 |
| BOL-57 | >300 | >300 | >300 | >300 | >300 |
| BOL-58 | >300 | >300 | >300 | >300 | >300 |
| BOL-59 | 75 | 200 | 200 | 100 | 150 |
| BOL-60 | 100 | 75 | 75 | 75 | 50 |
| BOL-61 | 18.75 | 25 | 37.5 | 75 | 6.25 |
| BOL-67 | 37.5 | 150 | 150 | 75 | 9.375 |
| BOL-68 | >300 | >300 | >300 | >300 | >300 |
| BOL-69 | 300 | >300 | >300 | 150 | >300 |
| BOL-70 | 150 | 200 | 300 | 100 | 100 |
| BOL-71 | >300 | (300) | >300 | >300 | >300 |
| BOL-72 | 75 | 50 | 50 | 75 | 75 |

(fortgesetzt)

| Mikroorganismen | 1 | 2 | 3 | 4 | 6 |
|---|---|---|---|---|---|
| BOL-73 | 37.5 | 37.5 | 75 b | 50 | 150 |
| BOL-74 | >300 | 300 | >300 | >300 | 75 |
| BOL-75 | 300 | 300 b | 300 | 200 | 300 |
| BOL-79 | 4.7 | >300 | >300 | 4.7 | <=1.56 |
| BOL-80 | >300 | >300 | >300 | >300 | >300 |
| BOL-81 | (300) | 200 | (75) | >300 | 50 |
| BOL-82 | 18.75 | 75 | 75 | 9.375 | 6.25 |
| BOL-83 | >300 | >300 | >300 | >300 | >300 |
| BOL-84 | >300 | >300 | >300 | >300 | 300 |
| BOL-85 | >300 | >300 | >300 | >300 | >300 |
| BOL-86 | (300) | >300 | (300) | >300 | 200 |
| BOL-87 | 37.5 | 37.5 | 37.5 | 25 | 37.5 |

Werte in Klammern bezeichnen eine 75%ige Inhibition des Mikroorganismenwachsstums bei der angegebenen Peptidkonzentration. Eine komplette Wachstumshemmung konnte in diesem Fall nicht erreicht werden.

Untersuchung der cytotoxischen Aktivität von Bolisinanaloga

Bolisinanaloga mit guter antimikrobieller Aktivität gegen mindestens einen der getesteten sieben Mikroorganismenstämme (MIC $\leq$ 37.5 $\mu$g/ml) wurden selektiert, um ihre cytotoxische Aktivität zu überprüfen.

Die Fähigkeit antimikrobieller Peptide, sich an Plasmamembranen anzulagern und diese zu permeabilisieren, wird in vielen Fällen als Wirkmechanismus dieser Substanzen angesehen. Es ist wünschenswert, dass bakterielle Membranen selektiv geschädigt werden, wohingegen die Zellen des Wirtes unbeeinflusst bleiben sollten. Als einfaches Modell zur Untersuchung der zytotoxischen Wirkung eines Peptids auf eukaryotische Zellen wird der Hämolysetest genutzt, bei dem die lysierende und somit toxische Aktivität der Peptide gegen rote Blutzellen (Erythrozyten) untersucht wird. Die Vorgehensweise zur Bestimmung der hämolytischen Aktivität der Peptide wurde in Anlehnung an Helmerhorst et al. (Helmerhorst et al., 1999, FEBS Lett. 449: 105-110) durchgeführt.

Erythrozyten wurden aus dem citrathaltigen Vollblut eines gesunden Probanden durch Zentrifugation (1500 $\times$ g, 20 °C, 10 min) isoliert und mit Testmedium 200-fach verdünnt. Peptide wurden in verschiedenen Konzentrationen in einer 96-Well-Mikrotiterplatte mit V-förmig zulaufendem Boden vorgelegt und für 1 h bei 37 °C mit der verdünnten Erythrozytensuspension inkubiert. Durch anschließende Zentrifugation (1000 x g für 5 min) wurden die Erythrozyten abgetrennt. Die durch freigesetztes Hämoglobin gefärbten Überstände wurden in eine 96-Well-Mikrotiterplatte mit flachem Boden überführt und ihre Absorption bei einer Wellenlänge von $\lambda$ =450 nm im Mikrotiterplatten-Leser bestimmt. Als Referenzwert für 100 %ige Hämolyse diente die Inkubation mit einer 1 %igen Lösung des Detergens Tween-20. Nur mit Testmedium inkubierte Erythrozyten dienten als Negativkontrolle.

Die Angabe der hämolytischen Aktivität von Bolisinanaloga erfolgte in Bezug auf die totale Hämolyse mit Tween-20 und wurde nach folgender Formel berechnet:

$$\text{Hämolyse [\%]} = [A_{450\,nm}(\text{Peptid}) - A_{450\,nm}(\text{Negativkontrolle})] / [A_{450\,nm}(1\%\ \text{Tween 20}) - A_{450\,nm}(\text{Negativkontrolle})] * 100$$

Als Testmedium diente TSB-Medium mit 287 mM Glukose. Die isotonische Glukosekonzentration soll einer unspezifischen Hämolyse im hypotonen Milieu vorbeugen (Osmoprotektion).

Es ist wünschenswert, dass die erfindungsgemässen Peptide keine oder nur moderate lytische Eigenschaften gegen Erythrozyten zeigen. Als moderate lytische Eigenschaften wird eine Lyse von weniger als 30 % der Erythrozyten bei Applikation von antimikrobiell wirksamen Peptidkonzentrationen bezeichnet.

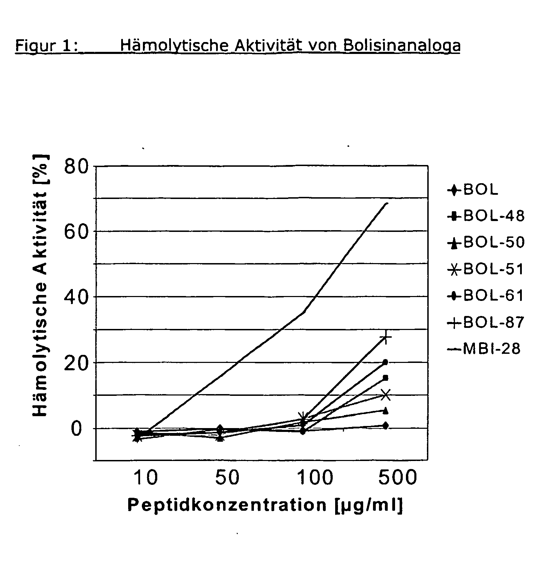

Figur 1 zeigt, daß Bolisinanaloga in antimikrobiell wirksamen Konzentrationen keine nennenswerte Hämolyse (< 5 %) bewirken. Auch in hohen Konzentrationen (500 μg/ml) ist nur eine moderate lytische Aktivität feststellbar (< 30 %). Zum Vergleich wurde die hämolytische Aktivität des antimikrobiellen Peptides MBI-28 (GOUGH et al., 1996, Infect. Immun. 64(12): 4922-7) aufgetragen.

**Patentansprüche**

1. Peptide mit antimikrobieller Aktivität der Aminosäuresequenz

$$R_1 - ZX_3X_3X_1BX_3RX_2X_3X_4BRX_2BX_3X_3B - R_2,$$

wobei

$R_1$ eine Aminogruppe (NH$_2$), eine Aminosäure oder ein Peptid mit bis zu 10 Aminosäuren und $R_2$ COOH, CONH$_2$, eine Aminosäure oder ein Peptid mit bis zu 10 Aminosäuren;
Z eine aromatische Aminosäure (W, Y, F), bevorzugt Tyrosin (Y);
$X_1$ Arginin (R) oder eine aromatische Aminosäure (W,Y,F), bevorzugt Tryptophan (W);
$X_2$ Serin (S), eine basische Aminosäure (R, K), bevorzugt Arginin (R), oder eine aromatische Aminosäure (W, Y,F), bevorzugt Tryptophan (W);
$X_3$ Threonin (T), eine hydrophobe Aminosäure (I, V, A, L) oder Arginin (R);
$X_4$ Asparaginsäure (D), Prolin (P), eine basische Aminosäure (K, R), bevorzugt Arginin (R) oder eine aromatische Aminosäure (W,Y,F), bevorzugt Tryptophan (W) und
B eine basische Aminosäure (K,R) ist,
sowie deren Derivate und/oder Fragmente mit antimikrobieller Aktivität, insbesondere die amidierten, acylierten, acetylierten, alkylierten, sulfatierten, phosphorylierten, halogenierten, insbesondere an aromatischen Aminosäureseitenketten halogenierten, glycosylierten, oxydierten, durch Veresterung oder Laktonformation modifizierten und/oder zyklisierten Derivate und/oder Fragmente
und wobei die Peptide nicht die Aminosäuresequenz
$R_3$-YIVYKIRSADKRSKALK-$R_4$ umfassen, worin $R_3$ eine Aminogruppe (NH$_2$), eine Aminosäure oder ein Peptid mit bis zu 50 Aminosäuren und $R_4$ COOH, CONH$_2$, eine Aminosäure oder ein Peptid mit bis zu 50 Aminosäuren darstellt, sowie nicht die amidierten, acetylierten, sulfatierten, phosphorylierten, glycosylierten, oxydierten Derivate und weiterhin Fragmente mit antimikrobiellen Eigenschaften, die aus der Aminosäuresequenz abgeleitet werden, insbesondere solche Fragmente, die durch konservativen Austausch oder Deletion von Aminosäuren erhalten wurden.

2. Peptide nach Anspruch 1 mit einer der Sequenzen

$R_1 - ZIX_3X_1BX_3RX_2ADBRX_2BALB - R_2$ ;
$R_1 - ZIX_3X_1BX_3RX_2APBRX_2BALB - R_2$ ;
$R_1 - ZIX_3X_1BX_3RX_2ABBRX_2BALB - R_2$ ;
$R_1 - ZIX_3X_1BX_3RX_2AZBRX_2BALB - R_2$ oder
$R_1 - ZIX_3X_1BX_3RX_2TZBRX_2BALB - R_2$

wobei $X_3$ eine hydrophobe Aminosäure (I, V) oder Arginin (R) ist und $R_1$, $R_2$, Z, $X_1$, $X_2$ und B die in Anspruch 1 angegebenen Bedeutungen haben,
sowie deren Derivate und/oder Fragmente mit antimikrobieller Aktivität, insbesondere die amidierten, acylierten, acetylierten, alkylierten, sulfatierten, phosphorylierten, halogenierten, insbesondere an aromatischen Aminosäureseitenketten halogenierten, glycosylierten, oxydierten, durch Veresterung oder Laktonformation modifizierten und/oder zyklisierten Derivate und/oder Fragmente.

3. Peptide nach Anspruch 1 und/oder 2 mit einer der Aminosäuresequenzen

| Seq ID NO | Sequenz |
|---|---|
| 1 | YIVYKIRSARKRSKALK |
| 2 | YIVYKIRSADKRRKALK |
| 3 | YIVYKRRSARKRSKALK |

(fortgesetzt)

| Seq ID NO | Sequenz |
|---|---|
| 4 | YIVYKRRSADKRRKALK |
| 5 | YIVYKIRSARKRRKALK |
| 6 | YIRYKIRSARKRRKALK |
| 7 | YIVYKRRSARKRRKALK |
| 8 | YIRYKRRSADKRRKALK |
| 9 | YIRYKIRSARKRRKALK |
| 10 | YIRYKRRSARKRRKALK |
| 11 | YIVYKIRSAPKRSKALK |
| 12 | YIVWKIRSADKRSKALK |
| 13 | YIVYKIRWADKRSKALK |
| 14 | YIVYKIRSAWKRSKALK |
| 15 | YIVYKIRSADKRWKALK |
| 16 | YIVYKIRSAWKRRKALK |
| 17 | YIVYKIRSAWKRWKALK |
| 18 | YIVYKIRSARKRWKALK |
| 19 | YIVYKIRWAWKRSKALK |
| 20 | YIVYKIRWAWKRRKALK |
| 21 | YIVYKIRWARKRSKALK |
| 22 | YIVYKIRWARKRRKALK |
| 23 | YIVYKIRWARKRWKALK |
| 24 | YIVYKIRRARKRRKALK |
| 25 | YIVYKIRRAWKRSKALK |
| 26 | YIVYKIRRAWKRRKALK |
| 27 | YIVYKIRRAWKRWKALK |
| 28 | YIVYKIRSAKKRKKALK |
| 29 | YIVYKIRSAKKRWKALK |
| 30 | YIVYKIRSAWKRKKALK |
| 31 | YIVYKIRWAKKRKKALK |
| 32 | YIVYKIRWAKKRWKALK |
| 33 | YIVYKIRWAWKRKKALK |
| 34 | YIVYKIRSAWKRWKAL |
| 35 | YIVYKIRSARKRWKAL |
| 36 | YIVYKIRSAWKRRK |
| 37 | YIVYKIRSAWKRWK |
| 38 | YIVYKIRSARKRWK |
| 39 | YIVYKIRWARKRRKAL |
| 40 | YIVYKIRWARKRRK |
| 41 | YIVWKIRSARKRRKALK |
| 42 | YIVWKIRSAWKRSKALK |
| 43 | YIVRKIRSAWKRWKALK |
| 44 | YIVRKIRSAWKRRKALK |
| 45 | YIVWKIRSARKRWKALK |
| 46 | YIVWKIRSAWKRWKALK |
| 47 | YIVRKIRSARKRRKALK |
| 48 | KYIVYKIRSARKRRKALK |
| 49 | KYIVYKIRSAWKRSKALK |
| 50 | KYIVYKIRSAWKRRKALK |
| 51 | KYIVYKIRWARKRRKALK |
| 52 | YIVYKIRFAFKRSKAL |
| 53 | YIVYKIRFAFKRRKAL |

(fortgesetzt)

| Seq ID NO | Sequenz |
|-----------|---------|
| 54 | YIVYKIRWAWKRSKAL |
| 55 | YIVYKIRWTWKRSKAL |
| 56 | KLAKRRKWAWRIKYVIY |

sowie deren Derivate und/oder Fragmente mit antimikrobieller Aktivität, insbesondere die amidierten, acylierten, acetylierten, alkylierten, sulfatierten, phosphorylierten, halogenierten, insbesondere an aromatischen Aminosäureseitenketten halogenierten, glycosylierten, oxydierten, durch Veresterung oder Laktonformation modifizierten und/oder zyklisierten Derivate und/oder Fragmente.

4. Peptide nach einem der Ansprüche 1 bis 3, worin eine oder mehrere Aminosäuren gegen die entsprechenden D-Aminosäuren ausgetauscht sind, Retro-, Inverso- oder Retro-Inverso-Peptide der Peptide nach einem der Ansprüche 1 bis 3.

5. Peptidmimetika der Peptide nach einem der Ansprüche 1 bis 4, die sich durch eine oder mehrere modifizierte Peptidbindungen, insbesondere durch reverse Peptidbindung oder durch Esterbindung auszeichnen.

6. Konjugate von Peptiden nach mindestens einem der Ansprüche 1 bis 5 mit Polymeren, vorzugsweise Polysorbat.

7. Nucleinsäuren, die für Peptide nach einem der Ansprüche 1 bis 4 kodieren.

8. Vektoren und Plasmide, die Nucleinsäuren nach Anspruch 7 enthalten.

9. Arzneimittel, enthaltend mindestens eines der Peptide gemäß Anspruch 1 bis 6, Nukleinsäuren nach Anspruch 7 oder Vektoren oder Plasmide nach Anspruch 8.

10. Arzneimittel nach Anspruch 9, enthaltend mindestens einen weiteren antibiotischen Wirkstoff.

11. Arzneimittel nach Anspruch 10, worin der antibiotische Wirkstoff der Gruppe der Penicilline, Cephalosporine, Carbacepheme, Cephamycine, Carbapeneme, Quinolone, Tetracycline, Aminoglycoside, Makrolide, Glycopeptide, Chloramphenicole, Glycylcycline, Licosamide, Fluoroquinolone und Peptidantibiotika entspricht.

12. Arzneimittel nach Anspruch 9 oder 10, enthaltend mindestens einen antiviralen, antiparasitischen und/oder antifungalen Wirkstoff.

13. Arzneimittel nach mindestens einem der Ansprüche 9 bis 12 in zur Applikation geeigneter Form, als Infusion, Salbe, Tablette, Spray oder "slow release"-Kapsel.

14. Diagnostikmittel, enthaltend mindestens eines der Peptide gemäß Anspruch 1 bis 6, Nukleinsäuren nach Anspruch 7 oder Vektoren oder Plasmide nach Anspruch 8.

15. Nahrungsmittel enthaltend Peptide gemäß mindestens einem der Ansprüche 1 bis 6.

16. Verfahren zur Inhibierung des Wachstums von Mikroorganismen, wobei die Mikroorganismen mit einer antimikrobiell wirksamen Menge eines oder mehrerer der Peptide entsprechend Anspruch 1 bis 6 in Kontakt gebracht werden.

17. Verfahren zur Herstellung der Peptide gemäß mindestens einem der Ansprüche 1 bis 6 durch Expression in prokaryontischen oder eukaryontischen Mikro-Organismen oder durch chemische Synthese.

18. Verwendung der Peptide nach mindestens einem der Ansprüche 1 bis 6 zur Herstellung eines Arzneimittels zur Behandlung von mikrobiell ausgelösten Erkrankungen, von Erkrankungen des menschlichen und tierischen Organismus, insbesondere mit Beteiligung des Magen-Darm-Traktes, der Atemwege und des Urogenitaltraktes, des Integumentes und seiner Anhangsdrüsen, zur Behandlung von chronischen Erkrankungen, zur Behandlung von durch mikrobielle Fehlbesiedlung bedingten Erkrankungen, verursacht durch Bakterien, Pilze, Hefen, Protisten, Viren, Mycoplasmen, Filarien und/oder Plasmodien, durch mikrobielle Fehlbesiedlungen hervorgerufene Infektionen, Entzündungen, mikrobiell induzierte Tumoren, mikrobiell bedingte degenerative Erkrankungen, Durchfallerkrankungen, Koliken, Abweichungen der Mund-, Darm- und Vaginalflora, Sepsis und/oder Karies sind.

19. Verwendung der Peptide nach mindestens einem der Ansprüche 1 bis 6 als Konservierungsstoff von Lebens- und Futtermitteln sowie Kosmetika, als Konservierungsstoff zur Vermeidung von mikrobiellen Kontaminationen von Medien und Gebrauchsgegenständen für die Zellkultur, als Desinfektionsmittel, zur Prävention von mikrobiellen Besiedelungen von Medizinprodukten, zur Prävention von mikrobiellen Besiedelungen von Bedarfsgegenständen, insbesondere Bekleidung und Schmuck, als Hilfsstoff bei industriellen Gärprozessen, insbesondere bei der Bierherstellung und bei der Joghurtproduktion.

**Claims**

1. Peptides having antimicrobial activity and comprising the amino acid sequence

$$R_1 - ZX_3X_3X_1BX_3RX_2X_3X_4BRX_2BX_3X_3B - R_2,$$

wherein

$R_1$ represents an amino group ($NH_2$), an amino acid or a peptide having up to 10 amino acids, and $R_2$ represents COOH, $CONH_2$, an amino acid or a peptide having up to 10 amino acids;
Z represents an aromatic amino acid (W, Y, F), preferably tyrosine (Y);
$X_1$ represents arginine (R) or an aromatic amino acid (W, Y, F), preferably tryptophan (W);
$X_2$ represents serine (S), a basic amino acid (R, K), preferably arginine (R), or an aromatic amino acid (W, Y, F), preferably tryptophan (W);
$X_3$ represents threonine (T), a hydrophobic amino acid (I, V, A, L) or arginine (R);
$X_4$ represents aspartic acid (D), proline (P), a basic amino acid (K, R), preferably arginine (R), or an aromatic amino acid (W, Y, F), preferably tryptophan (W); and
B represents a basic amino acid (K, R);
and their derivatives and/or fragments having antimicrobial activity, especially the derivatives and/or fragments which are amidated, acylated, acetylated, alkylated, sulfated, phosphorylated, halogenated, especially halogenated at aromatic amino acid side chains, glycosylated, oxidized, modified by esterification or lactone formation, and/or cyclized;
wherein said peptides do not have the amino acid sequence $R_3$-YIVYKIRSADKRSKALK-$R_4$, wherein $R_3$ is an amino group ($NH_2$), an amino acid or a peptide with up to 50 amino acids, and $R_4$ is COOH, $CONH_2$, an amino acid or a peptide with up to 50 amino acids, nor the amidated, acetylated, sulfated, phosphorylated, glycosylated, oxidized derivatives and further fragments having antimicrobial properties derived from this amino acid sequence, especially those fragments obtained by a conservative exchange or deletion of amino acids.

2. The peptides according to claim 1 having one of the following sequences:

$R_1$-ZIX$_3$X$_1$BX$_3$RX$_2$ADBRX$_2$BALB-R$_2$;
$R_1$-ZIX$_3$X$_1$BX$_3$RX$_2$APBRX$_2$BALB-R$_2$;
$R_1$-ZIX$_3$X$_1$BX$_3$RX$_2$ABBRX$_2$BALB-R$_2$;
$R_1$-ZIX$_3$X$_1$BX$_3$RX$_2$AZBRX$_2$BALB-R$_2$; or
$R_1$-ZIX$_3$X$_1$BX$_3$RX$_2$TZBRX$_2$BALB-R$_2$;

wherein $X_3$ represents a hydrophobic amino acid (I, V) or arginine (R), and $R_1$, $R_2$, Z, $X_1$, $X_2$ and B have the meanings as stated in claim 1, and their derivatives and/or fragments having antimicrobial activity, especially the derivatives and/or fragments which are amidated, acylated, acetylated, alkylated, sulfated, phosphorylated, halogenated, especially halogenated at aromatic amino acid side chains, glycosylated, oxidized, modified by esterification or lactone formation, and/or cyclized.

3. The peptides according to claim 1 and/or 2 having one of the following amino acid sequences:

| SEQ ID No.: | Sequence |
|---|---|
| 1 | YIVYKIRSARKRSKALK |

(continued)

| SEQ ID No.: | Sequence |
|---|---|
| 2 | YIVYKIRSADKRRKALK |
| 3 | YIVYKRRSARKRSKALK |
| 4 | YIVYKRRSADKRRKALK |
| 5 | YIVYKIRSARKRRKALK |
| 6 | YIRYKIRSARKRRKALK |
| 7 | YIVYKRRSARKRRKALK |
| 8 | YIRYKRRSADKRRKALK |
| 9 | YIRYKIRSARKRRKALK |
| 10 | YIRYKRRSARKRRKALK |
| 11 | YIVYKIRSAPKRSKALK |
| 12 | YIVWKIRSADKRSKALK |
| 13 | YIVYKIRWADKRSKALK |
| 14 | YIVYKIRSAWKRSKALK |
| 15 | YIVYKIRSADKRWKALK |
| 16 | YIVYKIRSAWKRRKALK |
| 17 | YIVYKIRSAWKRWKALK |
| 18 | YIVYKIRSARKRWKALK |
| 19 | YIVYKIRWAWKRSKALK |
| 20 | YIVYKIRWAWKRRKALK |
| 21 | YIVYKIRWARKRSKALK |
| 22 | YIVYKIRWARKRRKALK |
| 23 | YIVYKIRWARKRWKALK |
| 24 | YIVYKIRRARKRRKALK |
| 25 | YIVYKIRRAWKRSKALK |
| 26 | YIVYKIRRAWKRRKALK |
| 27 | YIVYKIRRAWKRWKALK |
| 28 | YIVYKIRSAKKRKKALK |
| 29 | YIVYKIRSAKKRWKALK |
| 30 | YIVYKIRSAWKRKKALK |
| 31 | YIVYKIRWAKKRKKALK |
| 32 | YIVYKIRWAKKRWKALK |
| 33 | YIVYKIRWAWKRKKALK |
| 34 | YIVYKIRSAWKRWKAL |
| 35 | YIVYKIRSARKRWKAL |
| 36 | YIVYKIRSAWKRRK |
| 37 | YIVYKIRSAWKRWK |
| 38 | YIVYKIRSARKRWK |
| 39 | YIVYKIRWARKRRKAL |
| 40 | YIVYKIRWARKRRK |
| 41 | YIVWKIRSARKRRKALK |
| 42 | YIVWKIRSAWKRSKALK |
| 43 | YIVRKIRSAWKRWKALK |
| 44 | YIVRKIRSAWKRRKALK |
| 45 | YIVWKIRSARKRWKALK |
| 46 | YIVWKIRSAWKRWKALK |
| 47 | YIVRKIRSARKRRKALK |
| 48 | KYIVYKIRSARKRRKALK |
| 49 | KYIVYKIRSAWKRSKALK |
| 50 | KYIVYKIRSAWKRRKALK |
| 51 | KYIVYKIRWARKRRKALK |

(continued)

| SEQ ID No.: | Sequence |
| --- | --- |
| 52 | YIVYKIRFAFKRSKAL |
| 53 | YIVYKIRFAFKRRKAL |
| 54 | YIVYKIRWAWKRSKAL |
| 55 | YIVYKIRWTWKRSKAL |
| 56 | KLAKRRKWAWRIKYVIY |

and their derivatives and/or fragments having antimicrobial activity, especially the derivatives and/or fragments which are amidated, acylated, acetylated, alkylated, sulfated, phosphorylated, halogenated, especially halogenated at aromatic amino acid side chains, glycosylated, oxidized, modified by esterification or lactone formation, and/or cyclized.

4. The peptides according to any of claims 1 to 3, wherein one or more amino acids are replaced by the corresponding D-amino acids, retro, inverso or retro-inverso peptides of the peptides according to any of claims 1 to 3.

5. Peptide mimetics of the peptides according to any of claims 1 to 4 which are **characterized by** one or more modified peptide bonds, especially by reverse peptide bonds or by ester bonds.

6. Conjugates of peptides according to at least one of claims 1 to 5 with polymers, preferably polysorbate.

7. Nucleic acids coding for peptides according to any of claims 1 to 4.

8. Vectors or plasmids containing nucleic acids according to claim 7.

9. A medicament containing at least one of the peptides according to claims 1 to 6, nucleic acids according to claim 7 or vectors or plasmids according to claim 8.

10. The medicament according to claim 9, containing at least one further antibiotically effective ingredient.

11. The medicament according to claim 10, wherein said antibiotically effective ingredient corresponds to the group of penicillins, cephalosporins, carbacephems, cephamycins, carbapenems, quinolones, tetracyclines, aminoglycosides, macrolides, glycopeptides, chloroamphenicols, glycylcyclines, licosamides, fluoroquinolones and peptide antibiotics.

12. The medicament according to claim 9 or 10, containing at least one antivirally, antiparasitically and/or antifungally effective ingredient.

13. The medicament according to at least one of claims 9 to 12 in a form suitable for application, as an infusion, ointment, tablet, spray or "slow release" capsule.

14. A diagnostic agent containing at least one of the peptides according to claims 1 to 6, nucleic acids according to claim 7 or vectors or plasmids according to claim 8.

15. A foodstuff containing peptides according to at least one of claims 1 to 6.

16. A method for inhibiting the growth of microorganisms, wherein said microorganisms are contacted with an antimicrobially effective amount of one or more of the peptides according to claims 1 to 6.

17. A process for preparing the peptides according to at least one of claims 1 to 6 by expression in prokaryotic or eukaryotic microorganisms or by chemical synthesis.

18. Use of the peptides according to at least one of claims 1 to 6 for preparing a medicament for the treatment of microbially caused diseases, of diseases of the human and animal organism, especially those involving the gastrointestinal tract, the respiratory tract and the urogenital tract, the integument and its appendage glands, for the treatment of chronic diseases, for the treatment of diseases caused by misplaced microbial colonizations caused by bacteria, fungi, yeasts, protists, viruses, mycoplasmas, filariae and/or plasmodiums, infections caused by misplaced microbial colonizations, inflammations, microbially induced tumors, microbially caused degenerative diseases, diarrheic diseases, colics, deviations in the oral, intestinal and vaginal floras, sepsis and/or caries.

19. Use of the peptides according to at least one of claims 1 to 6 as a preservative for foodstuffs and fodders as well as cosmetics, as a preservative for avoiding microbial contaminations of media and utility articles for cell culture, as a disinfectant, for the prevention of microbial colonizations on medical products, for the prevention of microbial colonizations on requisites, such as clothing and jewels, as an auxiliary agent in industrial fermentation processes, especially in beer production and yoghurt production.

**Revendications**

1. Peptides ayant une activité antimicrobienne, qui présentent la séquence d'acides aminés $R_1$ - $ZX_3X_3X_1BX_3RX_2X_3X_4BRX_2BX_3X_3B$ - $R_2$,
   dans laquelle

   - $R_1$ représente un groupe amino ($NH_2$), un acide aminé ou un peptide contenant jusqu'à 10 acides aminés et $R_2$ représente COOH, $CONH_2$, un acide aminé ou un peptide contenant jusqu'à 10 acides aminés ;
   - Z représente un acide aminé aromatique (W, Y, F), de préférence la tyrosine (Y) ;
   - $X_1$ représente l'arginine (R) ou un acide aminé aromatique (W,Y,F), de préférence le tryptophane (W) ;
   - $X_2$ représente la sérine (S), un acide aminé basique (R, K), de préférence l'arginine (R), ou un acide aminé aromatique (W,Y,F), de préférence le tryptophane (W) ;
   - $X_3$ représente la thréonine (T), un acide aminé hydrophobe (I, V, A, L) ou l'arginine (R) ;
   - $X_4$ représente l'acide aspartique (D), la proline (P), un acide aminé basique (K, R), de préférence l'arginine (R) ou un acide aminé aromatique (W,Y,F), de préférence le tryptophane (W) et
   - B représente un acide aminé basique (K, R),

   ainsi que leurs dérivés et/ou fragments ayant une activité antimicrobienne, en particulier les dérivés et/ou fragments amidés, acylés, acétylés, alkylés, sulfatés, phosphorylés, halogénés, en particulier halogénés, glycosylés, oxydés, modifiés par estérification ou formation d'une lactone et/ou cyclisés au niveau des chaînes latérales aromatiques d'acide aminé et dans laquelle les peptides ne comprennent pas la séquence d'acides aminés $R_3$-YIVYKIRSADKRSKALK-$R_4$, dans laquelle $R_3$ représente un groupe amino ($NH_2$), un acide aminé ou un peptide contenant jusqu'à 50 acides aminés et $R_4$ représente COOH, $CONH_2$, un acide aminé ou un peptide contenant jusqu'à 50 acides aminés, mais pas les dérivés amidés, acétylés, sulfatés, phosphorylés, glycosylés, oxydés ainsi que les fragments ayant des propriétés antimicrobiennes, qui sont dérivés de la séquence d'acides aminés, en particulier les fragments, qui sont obtenus au moyen d'une substitution conservative ou d'une délétion d'acides aminés.

2. Peptides selon la revendication 1 présentant l'une des séquences

   - $R_1$ - $ZIX_3X_1BX_3RX_2ADBRX_2BALB$ - $R_2$ ;
   - $R_1$ - $ZIX_3X_1BX_3RX_2APBRX_2BALB$ - $R_2$ ;
   - $R_1$ - $ZIX_3X_1BX_3RX_2ABBRX_2BALB$ - $R_2$ ;
   - $R_1$ - $ZIX_3X_1BX_3RX_2AZBRX_2BALB$ - $R_2$ ou
   - $R_1$ - $ZIX_3X_1BX_3RX_2TZBRX_2BALB$ - $R_2$

   dans lesquelles $X_3$ représente un acide aminé hydrophobe (I, V) ou l'arginine (R) et $R_1$, $R_2$, Z, $X_1$, $X_2$ et B ont les significations indiquées à la revendication 1,
   ainsi que leurs dérivés et/ou fragments ayant une activité antimicrobienne, en particulier les dérivés et/ou fragments amidés, acylés, acétylés, alkylés, sulfatés, phosphorylés, halogénés, en particulier les dérivés et/ou fragments halogénés, glycosylés, oxydés, modifiés par estérification ou formation d'une lactone et/ou cyclisés au niveau des chaînes latérales aromatiques d'acide aminé.

3. Peptides selon la revendication 1 et/ou 2 présentant l'une des séquences d'acides aminés parmi

| SEQ ID N° | Séquence |
|---|---|
| 1 | YIVYKIRSARKRSKALK |
| 2 | YIVYKIRSADKRRKALK |
| 3 | YIVYKRRSARKRSKALK |
| 4 | YIVYKRRSADKRRKALK |
| 5 | YIVYKIRSARKRRKALK |
| 6 | YIRYKIRSARKRRKALK |
| 7 | YIVYKRRSARKRRKALK |
| 8 | YIRYKRRSADKRRKALK |
| 9 | YIRYKIRSARKRRKALK |
| 10 | YIRYKRRSARKRRKALK |
| 11 | YIVYKIRSAPKRSKALK |
| 12 | YIVWKIRSADKRSKALK |
| 13 | YIVYKIRWADKRSKALK |
| 14 | YIVYKIRSAW KRSKALK |
| 15 | YIVYKIRSADKRW KALK |
| 16 | YIVYKIRSAWKRRKALK |
| 17 | YIVYKIRSAWKRWKALK |
| 18 | YIVYKIRSARKRWKALK |
| 19 | YIVYKIRWAWKRSKALK |
| 20 | YIVYKIRWAWKRRKALK |
| 21 1 | YIVYKIRWARKRSKALK |
| 22 | YIVYKIRWARKRRKALK |
| 23 | YIVYKIRWARKRWKALK |
| 24 | YIVYKIRRARKRRKALK |
| 25 | YIVYKIRRAWKRSKALK |
| 26 | YIVYKIRRAWKRRKALK |
| 27 | YIVYKIRRAWKRWKALK |
| 28 | YIVYKIRSAKKRKKALK |
| 29 | YIVYKIRSAKKRWKALK |
| 30 | YIVYKIRSAWKRKKALK |
| 31 | YIVYKIRWAKKRKKALK |
| 32 | YIVYKIRWAKKRWKALK |
| 33 | YIVYKIRWAWKRKKALK |
| 34 | YIVYKIRSAWKRWKAL |
| 35 | YIVYKIRSARKRWKAL |
| 36 | YIVYKIRSAWKRRK |
| 37 | YIVYKIRSAWKRWK |
| 38 | YIVYKIRSARKRWK |
| 39 | YIVYKIRWARKRRKAL |
| 40 | YIVYKIRWARKRRK |
| 41 | YIVWKIRSARKRRKALK |
| 42 | YIVWKIRSAWKRSKALK |
| 43 | YIVRKIRSAWKRWKALK |
| 44 | YIVRKIRSAWKRRKALK |
| 45 | YIVWKIRSARKRWKALK |
| 46 | YIVWKIRSAWKRWKALK |
| 47 | YIVRKIRSARKRRKALK |
| 48 | KYIVYKIRSARKRRKALK |
| 49 | KYIVYKIRSAWKRSKALK |
| 50 | KYIVYKIRSAWKRRKALK |

(suite)

| SEQ ID N° | Séquence |
|---|---|
| 51 | KYIVYKIRWARKRRKALK |
| 52 | YIVYKIRFAFKRSKAL |
| 53 | YIVYKIRFAFKRRKAL |
| 54 | YIVYKIRWAWKRSKAL |
| 55 | YIVYKIRWTWKRSKAL |
| 56 | KLAKRRKWAW RIKYVIY |

ainsi que leurs dérivés et/ou fragments ayant une activité antimicrobienne, en particulier les dérivés et/ou fragments amidés, acylés, acétylés, alkylés, sulfatés, phosphorylés, halogénés, en particulier halogénés, glycosylés, oxydés, modifiés par estérification ou formation d'une lactone et/ou cyclisés au niveau des chaînes latérales aromatiques d'acide aminé.

4. Peptides selon l'une quelconque des revendications 1 à 3, dans lesquels un ou plusieurs acides aminés sont substitués par les acides D-aminés correspondants, rétro-, inverso- ou rétro-inverso-peptides des peptides selon l'une quelconque des revendications 1 à 3.

5. Peptidomimétiques des peptides selon l'une quelconque des revendications 1 à 4, qui se caractérisent par une ou plusieurs liaisons peptidiques modifiées, en particulier par une liaison peptidique inversée ou par une liaison ester.

6. Conjugués de peptides selon au moins l'une quelconque des revendications 1 à 5 avec des polymères, de préférence un polysorbate.

7. Acides nucléiques qui codent pour les peptides selon l'une quelconque des revendications 1 à 4.

8. Vecteurs et plasmides qui contiennent les acides nucléiques selon la revendication 7.

9. Médicament contenant au moins l'un des peptides selon les revendications 1 à 6, les acides nucléiques selon la revendication 7 ou les vecteurs ou plasmides selon la revendication 8.

10. Médicament selon la revendication 9 contenant au moins un autre principe actif antibiotique.

11. Médicament selon la revendication 10, dans lequel le principe actif antibiotique est issu du groupe des pénicillines, des céphalosporines, des carbacéphèmes, des céphamycines, des carbapénèmes, des quinolones, des tétracyclines, des aminoglycosides, des macrolides, des glycopeptides, des chloramphénicols, des glycylcyclines, des licosamides, des fluoroquinolones et des antibiotiques peptidiques.

12. Médicament selon la revendication 9 ou 10 contenant au moins un principe actif antiviral, antiparasitaire et/ou antifongique.

13. Médicament selon au moins l'une quelconque des revendications 9 à 12 sous la forme appropriée pour une application, sous forme de perfusion, pommade, comprimé, fluide à pulvériser ou capsule à libération prolongée.

14. Agent diagnostique contenant au moins l'un des peptides selon les revendications 1 à 6, les acides nucléiques selon la revendication 7 ou les vecteurs ou plasmides selon la revendication 8.

15. Produit alimentaire contenant les peptides selon au moins l'une quelconque des revendications 1 à 6.

16. Procédé d'inhibition de la croissance de microorganismes, dans lequel les microorganismes sont mis en contact avec une quantité efficace sur le plan antimicrobien d'un ou plusieurs des peptides selon les revendications 1 à 6.

17. Procédé de préparation des peptides selon au moins l'une quelconque des revendications 1 à 6 au moyen d'une expression dans des micro-organismes procaryotes ou eucaryotes ou d'une synthèse chimique.

18. Utilisation des peptides selon au moins l'une quelconque des revendications 1 à 6 pour la fabrication d'un médicament destiné au traitement de maladies d'origine microbienne, de maladies de l'organisme humain et animal, dans lesquelles sont impliqués en particulier le tractus gastro-intestinal, les voies respiratoires et le tractus uro-génital, du tégument et de ses glandes associées, destiné au traitement de maladies chroniques, destiné au traitement de maladies dues à une prolifération microbienne provoquée par des bactéries, des champignons, des levures, des protistes, des virus, des mycoplasmes, des filaires et/ou des plasmodies, destiné au traitement d'infections dues à une prolifération microbienne, d'inflammations, de tumeurs induites par voie microbienne, de maladies dégénératives induites par voie microbienne, de diarrhées, de coliques, de modifications de la flore buccale, intestinale et vaginale, de la septicémie et/ou des caries.

19. Utilisation des peptides selon au moins l'une quelconque des revendications 1 à 6 en tant qu'agent conservateur pour des produits alimentaires et des aliments pour animaux ainsi que pour des produits cosmétiques, en tant qu'agent conservateur pour éviter les contaminations microbiennes de milieux et d'ustensiles pour la culture de cellules, en tant que produit désinfectant, pour la prévention de la prolifération microbienne dans des produits médicaux, pour la prévention de la prolifération microbienne dans des objets usuels, en particulier les vêtements et les bijoux, en tant qu'adjuvant pour des procédés de fermentation industriels, en particulier pour la fabrication de la bière et la production de yaourt.

Figur 1: Hämolytische Aktivität von Bolisinanaloga

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9735877 A
- EP 0106518 W

- DE 4444753 A1

**In der Beschreibung aufgeführte Nicht-Patentliteratur**

- **HELMERHORST et al.** *FEBS Lett.,* 1999, vol. 449, 105-110

- **GOUGH et al.** *Infect. Immun.,* 1996, vol. 64 (12), 4922-7